# FASCICULE DE BREVET EUROPEEN

(11) **EP 2 079 646 B1**
(45) Date de publication et mention de la délivrance du brevet: **09.03.2016**
(21) Numéro de dépôt: 07858580.9
(22) Date de dépôt: 15.10.2007
(51) Int. Cl.: B65D 83/14

(54) **DISPOSITIF DE DISTRIBUTION DE PRODUIT FLUIDE**
SPENDER FÜR EIN FLÜSSIGPRODUKT
FLUID PRODUCT DISPENSING DEVICE

(30) Priorité: 20.10.2006 FR 0654402
(43) Date de publication de la demande: 22.07.2009
(73) Titulaire: Aptar France SAS, 27110 Le Neubourg (FR)
(72) Inventeur: MARGHERITIS, Antonio, 21033 Cittiglio (IT)
(74) Mandataire: CAPRI
(86) Numéro de dépôt international: PCT/FR2007/052153
(87) Numéro de publication internationale: WO 2008/047035

(56) Documents cités:
- EP-A- 1 108 477
- FR-A- 1 447 222
- FR-A- 2 060 343
- JP-A- 2006 240 638
- US-A1- 2004 139 964
- US-B1- 6 352 181

## Description

La présente invention concerne un dispositif de distribution de produit fluide, et plus particulièrement un tel dispositif comportant un élément de blocage permettant d'empêcher l'actionnement dudit dispositif entre deux utilisations.

Les systèmes qui servent à empêcher l'actionnement d'un dispositif de distribution de produit fluide entre deux actionnements sont bien connus, et comportent généralement des dispositifs amovibles, tels que des clips que l'on vient fixer sous la tête d'actionnement ou poussoir pour empêcher son déplacement. L'inconvénient d'un tel dispositif amovible est qu'il peut parfois se détacher de lui-même pendant son transport dans un sac par exemple. Il existe des systèmes intégrés dans le dispositif et qui ne sont pas amovibles mais ces systèmes sont généralement très compliqués à réaliser et à assembler, et impliquent une modification de plusieurs pièces du dispositif de distribution, ce qui en augmente considérablement le coût. les documents US 6352181 et JP 2006 240638 décrivent des dispositifs de l'art antérieur. US 6352181 décrit un dispositif selon le préamble de la revendication 1.

La présente invention a pour but de fournir un dispositif de distribution de produit fluide qui ne reproduit pas les inconvénients susmentionnés.

Plus particulièrement, la présente invention a pour but de fournir un dispositif de distribution de produit fluide comportant un élément de blocage d'actionnement qui soit simple et peu coûteux à fabriquer et à assembler.

La présente invention a également pour but de fournir un tel dispositif de distribution de produit fluide qui s'adapte facilement sur les dispositifs existants et ne nécessite pas de modification des pièces constitutives du dispositif de distribution.

La présente invention a aussi pour but de fournir un tel dispositif qui permette de visualiser simplement si le dispositif a déjà été utilisé ou non.

La présente invention a également pour but de fournir un tel dispositif dont le fonctionnement est sûr et fiable, et dont l'actionnement est aisé pour l'utilisateur.

La présente invention a donc pour objet un dispositif de distribution de produit fluide, comprenant un organe de distribution de produit fluide, tel qu'une pompe ou une valve actionnée manuellement, ledit organe de distribution de produit fluide étant fixé par une bague de fixation sur un réservoir, une tête d'actionnement, montée sur ledit organe de distribution et déplaçable pour actionner ledit organe de distribution, un élément de blocage amovible qui, en position de blocage empêche l'actionnement de l'organe de distribution, au moins un élément sécable solidaire de l'élément de blocage pour témoigner d'une première utilisation, ledit élément de blocage étant assemblé autour de la bague de fixation, entre la tête et le réservoir, et coopère, en position de blocage, avec ladite tête d'actionnement et ledit réservoir pour empêcher une déplacement axial de ladite tête d'actionnement par rapport audit réservoir, ledit au moins un élément sécable, avant la première utilisation, entourant au moins partiellement ledit élément de blocage.

Avantageusement, ledit élément sécable est clipsé sur ledit élément de blocage lors de son assemblage.

Avantageusement, ledit élément de blocage comporte au moins une ouverture, adaptée à recevoir au moins un élément d'enclipsage dudit élément sécable.

Avantageusement, l'élément de blocage comporte au moins une projection s'étendant radialement vers l'intérieur dudit élément de blocage.

Avantageusement, l'élément de blocage comporte une bague ouverte élastiquement déformable.

Avantageusement, l'élément de blocage et ledit au moins un élément sécable forment une bague fermée, avant la première utilisation.

Avantageusement, ledit au moins un élément sécable est adapté à se casser lorsqu'il est séparé de l'élément de blocage.

Avantageusement, ledit au moins un élément sécable est formé par une barrette reliant les deux côtés de ladite bague ouverte, avant la première utilisation.

Avantageusement, ladite barrette, avant la première utilisation est, au niveau d'une première extrémité, solidaire d'un premier desdits deux côtés de ladite bague ouverte, s'étend autour de la partie de la paroi latérale de ladite bague de fixation non encerclée par ladite bague ouverte, et coopère, au niveau d'une deuxième extrémité, avec le deuxième desdits deux côtés de ladite bague ouverte.

Avantageusement, ladite barrette se casse au niveau de la liaison solidaire entre ladite barrette et ladite bague ouverte.

Avantageusement, l'élément de blocage est maintenu en position de blocage par des moyens de retenue, lesdits moyens de retenue étant déformables élastiquement.

Avantageusement, lesdits moyens de retenue sont formés par ladite bague ouverte et/ou au moins une projection de blocage.

Avantageusement, ledit élément de blocage comporte des moyens de préhension pour l'encliquetage et le désencliquetage de ladite bague ouverte.

D'autres caractéristiques et avantages apparaîtront au cours de la description détaillée suivante d'un mode de réalisation avantageux de la présente invention, faite en référence aux dessins joints, donnés à titre d'exemples non limitatifs, et sur lesquels :

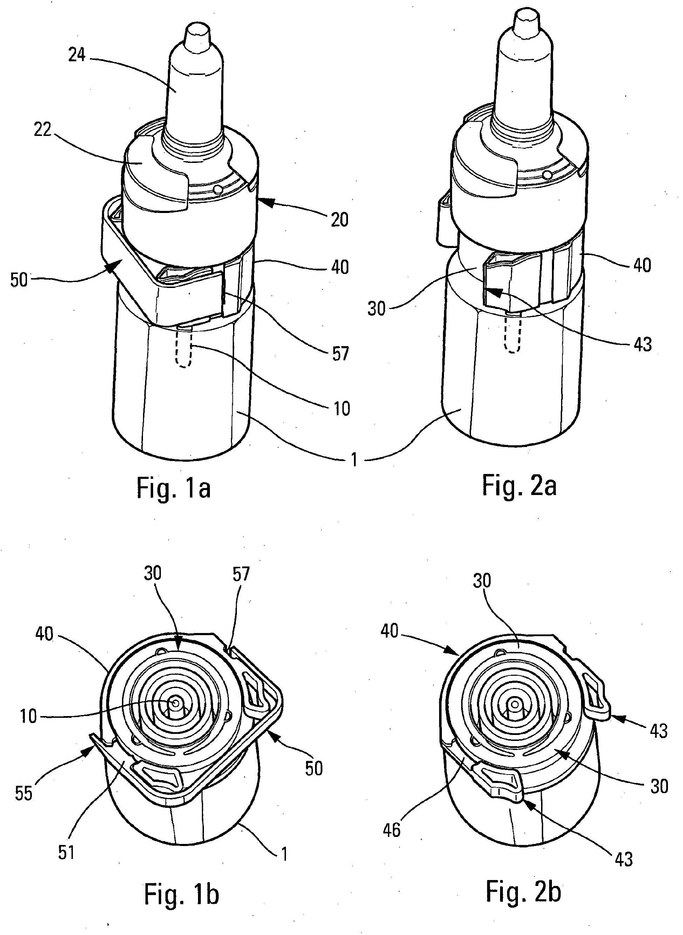

Les figures 1a et 2a représentent des vues schématiques en perspective d'un dispositif de distribution de produit fluide respectivement en position de blocage avant la première utilisation et en position de blocage après la première utilisation.
Les figures 1b et 2b représentent des vues schématiques en coupe horizontale, respectivement des figures 1a et 2a.
La figure 3 est une vue en section horizontale du dispositif, avant l'assemblage de l'élément de blocage sur la bague de fixation.
La figure 4 est une vue similaire à la figure 3, une fois l'élément de blocage assemblé sur la bague de fixation.
La figure 5 est une vue similaire à la figure 4, montrant le dispositif de distribution, une fois l'élément sécable enclipsé sur l'élément de blocage.
La figure 6 est une vue similaire à la figure 5, en position de blocage, une fois l'élément sécable séparé de l'élément de blocage, après une première utilisation.
La figure 7 est une vue schématique de côté du dispositif de distribution de produit fluide, en position de blocage.
La figure 8 est une vue schématique en section transversale de la figure 7.
La figure 9 est une vue schématique en perspective du dispositif de distribution en position de blocage, laissant apparaître l'ouverture d'enclipsage de l'élément de blocage, l'élément sécable ayant déjà été déclipsé.

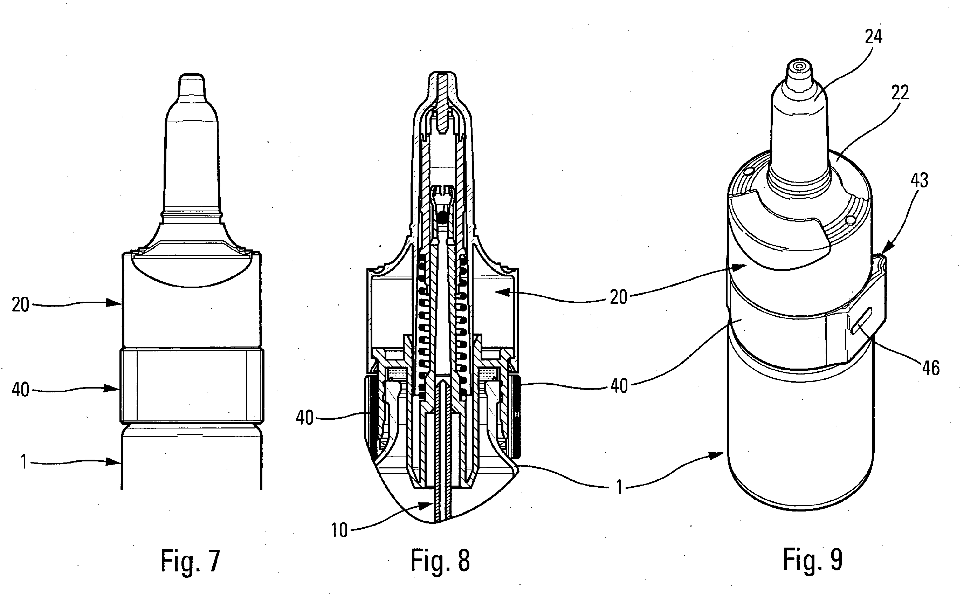

En référence aux figures, le dispositif de distribution comporte un organe de distribution 10 qui, dans l'exemple représenté sur les figures, est une pompe. L'invention s'applique bien sûr à tous types d'organes de distribution, et notamment aux valves. La pompe peut être assemblée sur un réservoir 1 au moyen d'une bague de fixation 30, qui peut être quelconque, et notamment vissable, sertissable ou encliquetable. La bague 30 peut être un élément séparé, ou être réalisé d'une pièce monobloc avec une partie du dispositif, tel qu'un piston de la pompe, comme représenté sur la figure 8. Le corps de pompe pourrait aussi être monobloc avec le réservoir, auquel cas la partie de liaison monobloc entre le corps de pompe et le réservoir pourrait agir en tant que bague de fixation au sens de la présente invention. Un poussoir ou tête d'actionnement 20 est assemblé sur la pompe pour actionner la pompe. Dans l'exemple représenté sur les figures, la tête d'actionnement est un poussoir nasal qui comporte une partie longitudinale 24 destinée à pénétrer dans la narine et une zone d'appui 22 sur laquelle l'utilisateur appuie pour actionner le dispositif. Il est entendu que l'exemple de réalisation représenté sur les figures, notamment le type de pompe ou le type de poussoir n'est absolument pas limitatif, et-que la présente invention peut au contraire s'appliquer à tous types de dispositifs de distribution de produit fluide.

Selon l'invention, le dispositif comporte un élément de blocage d'actionnement 40 qui est amovible sur le dispositif entre une position de blocage dans laquelle il empêche l'actionnement de la pompe, et une position retirée, dans laquelle l'élément de blocage 40 se trouve séparé du dispositif de distribution, ce qui permet l'actionnement de la pompe. L'élément de blocage 40 est, en position de blocage, assemblé autour de la bague de fixation 30 entre la tête d'actionnement 20 et le réservoir 1, comme représenté sur les figures. Cet exemple n'est pas limitatif et la bague pourrait aussi coopérer avec un autre élément, qui coopèrerait avantageusement à son tour avec l'élément de blocage 40. Le but de l'élément de blocage, en position de blocage, est d'empêcher un déplacement de la tête par rapport à la pompe, bloquant ainsi l'actionnement de celle-ci. L'élément de blocage est avantageusement déplaçable transversalement à l'axe longitudinal de l'organe de distribution 10, comme représenté sur les figures. Au moins un moyen de préhension 43 de l'élément de blocage permet à l'utilisateur de mettre en place et/ou de retirer aisément l'élément de blocage autour de la bague de fixation. Les moyens de préhension peuvent consister en des bords plus épais par rapport à la structure de l'élément de blocage. Comme représenté sur les figures, il peut s'agir d'un bord à double parois latérales, dépassant de l'élément de blocage et donc facilement accessible par l'utilisateur.

L'élément de blocage comporte des moyens témoins de premier usage, comprenant au moins un élément sécable 50. Les figures montrent un exemple avec un seul élément sécable, mais on pourrait en prévoir plusieurs. Cet élément sécable, en position de blocage avant la première utilisation, entoure au moins partiellement l'élément de blocage. Lors de la première utilisation, cet élément sécable est adapté à se casser et à se séparer de l'élément de blocage.

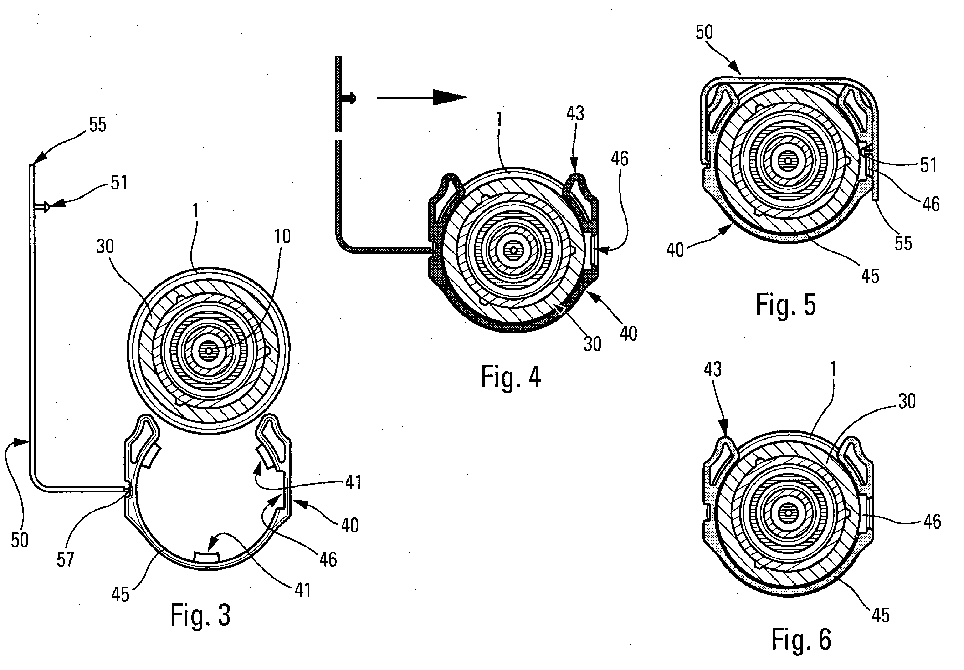

Comme représenté sur la figure 3, l'élément de blocage peut comporter au moins une projection 41. Ladite au moins une projection s'étend radialement vers l'intérieur depuis la paroi latérale interne de l'élément de blocage. Ainsi lorsque l'élément de blocage 40 se trouve en position de blocage autour de la bague de fixation 30, ladite au moins une projection peut s'insérer entre un bord inférieur de la bague de fixation 30 et le réservoir 1. Ladite projection peut alors servir à caler l'élément de blocage autour de la bague de fixation et ainsi éviter tout déplacement de l'élément de blocage lorsqu'il est en position de blocage. Ladite au moins une projection 41 peut aussi coopérer avec d'autres parties constitutives du dispositif.

L'élément de blocage, selon le mode de réalisation représenté sur les figures, comporte avantageusement une bague ouverte 45 élastiquement déformable. La figure 3 représente la bague ouverte avant son montage sur la bague de fixation 30. Cette bague ouverte peut comprendre trois projections 41, disposées par exemple de manière symétrique par rapport à l'axe longitudinal du dispositif. Les extrémités libres de la bague ouverte sont chacune pourvue d'un moyen de préhension 43 sous la forme d'une double paroi latérale de forme sensiblement arrondie, venant s'encliqueter facilement autour de la bague de fixation. Le moyen de préhension pourrait aussi être prévu en un autre endroit, par exemple au milieu de la bague ouverte. D'un côté de la paroi latérale de la bague ouverte 45 se trouve une liaison solidaire 57 de l'élément sécable 50, (avant la première utilisation du dispositif), et de l'autre côté de la paroi latérale de la bague ouverte se trouve au moins une ouverture 46 destinée à accueillir l'élément d'enclipsage 51 de l'élément sécable.

L'élément de blocage peut aussi comporter des moyens de retenue servant à maintenir la bague ouverte autour de la bague de fixation. Ces moyens de retenue sont déformables élastiquement pour permettre l'encliquetage et le désencliquetage de la bague ouverte autour de la bague de fixation. Lesdits moyens de retenue peuvent être réalisés par l'élasticité de la bague ouverte 45 elle-même, ou bien il peut s'agir des projections 41. Ces moyens de retenue sont particulièrement utiles, une fois l'élément sécable déclipsé de l'élément de blocage, afin de retenir ce dernier en position de blocage entre les différentes utilisations du dispositif de distribution.

L'élément sécable est solidaire de l'élément de blocage au niveau de la liaison solidaire 57 située sur la paroi externe de la bague ouverte, comme représenté sur les figures 3 à 5. L'élément sécable s'étend au moins partiellement autour de la bague de fixation, rejoignant par son extrémité libre 55 la paroi latérale de l'autre côté de la bague ouverte. Cette extrémité libre de l'élément sécable 50 comporte un élément d'enclipsage 51, pouvant être par exemple une tête d'épingle ou similaire, venant en prise avec une ouverture correspondante 46 située sur ledit autre côté de la bague ouverte.

Ainsi, après l'assemblage initial de l'élément de blocage 40 et de l'élément sécable 50 enclipsé, autour de la bague de fixation 30, le dispositif de distribution est bloqué en position de blocage par une bague fermée, celle-ci étant constituée par la bague ouverte 45 dont les extrémités sont reliées par l'intermédiaire de l'élément sécable 50. L'élément sécable peut être réalisé en un matériau suffisamment flexible pour s'adapter et se positionner étroitement d'une part contre les bords 43 de la bague ouverte, et d'autre part contre la partie de la paroi latérale de la bague de fixation qui ne se trouve pas encerclée par ladite bague ouverte. Seul le bord 55 de l'extrémité libre enclispée dépasse légèrement du système de bague fermée pour constituer un moyen de déclispage 55 facilement accessible par l'utilisateur et permettre un déclipsage et une séparation pratique de l'élément sécable lors de la première utilisation.

Avantageusement, l'élément de blocage, y compris l'élément sécable, est réalisé en matériau plastique à la fois résistant pour permettre à l'élément de blocage d'être réutilisable, et déformable pour permettre l'encliquetage et le désencliquetage de l'élément de blocage ainsi que l'adaptation de l'élément sécable au cours de l'assemblage à la forme de l'élément de blocage et de la bague de fixation.

Selon un mode de réalisation avantageux, l'élément sécable forme une barrette s'étendant d'un côté de la bague ouverte, puis le long de la paroi latérale de ce côté de la bague ouverte en direction du bord de la bague, s'étendant ensuite autour de la partie de la paroi latérale de ladite bague de fixation non encerclée par la bague ouverte, puis rejoignant le bord de l'autre côté de la bague ouverte, l'extrémité libre de ladite barrette s'enclipsant ainsi dans l'ouverture de ce même côté, tout en continuant sa trajectoire autour de la bague ouverte pour laisser dépasser son bord libre, qui constitue le moyen de déclispage 55.

Lors de la première utilisation, le déclipsage de l'élément sécable 50 entraîne, de préférence automatiquement, sa séparation de la bague ouverte 40 au niveau de sa liaison solidaire 57 avec celle-ci. Il reste ensuite, pour l'utilisateur, à désencliqueter l'élément de blocage 40 de la bague de fixation 30 pour mettre le dispositif de distribution en position de distribution et permettre l'actionnement de la pompe. La présence d'une barrette 50 enclipsée et intacte sur l'élément de blocage garantit donc à l'utilisateur que l'élément de blocage 40 n'a encore jamais été déplacé hors de sa position de blocage. Ainsi il est certain que le dispositif n'a encore jamais été actionné.

Après la première utilisation, la bague peut être remise en place en position de blocage. Il suffit à l'utilisateur d'encliqueter la bague ouverte autour de la bague de fixation. L'encliquetage est facilité par la forme des bords des deux extrémités de la bague ouverte, au niveau des moyens de préhension 43. Comme le montre la figure 3, les deux bords des moyens de préhension, mis au contact de la bague de fixation, décrivent chacun une tangente par rapport à la paroi circulaire de la bague de fixation, pour permettre un engagement par glissement, plus aisé, des extrémités de la bague autour de la tête jusqu'à l'encliquetage. La forme des bords des extrémités de la bague présente l'autre avantage de ne pas endommager la tête d'actionnement au cours des encliquetages et désencliquetages successifs de chaque utilisation ultérieure du dispositif de distribution. Une fois encliquetée, la bague ouverte 45 s'étend ainsi de nouveau au moins partiellement autour de la tête de fixation 30, chaque projection de blocage 41 coopérant avec la bague de fixation 30 ou un élément solidaire de celle-ci.

Avantageusement, l'élément de blocage 40 selon l'invention peut être engagé sur la bague de fixation 30 par n'importe quel côté. Ce mode de réalisation permet un assemblage simple et rapide car il ne nécessite pas d'orienter chaque bague de fixation, lors du montage de l'élément de blocage sur la bague de fixation. De même, pour l'utilisateur, la remise en place de l'élément de blocage peut se faire très facilement après chaque utilisation.

Avantageusement, l'élément de blocage de la présente invention, quels que soient les modes de réalisation, s'applique à tous les dispositifs de distribution de produit fluide existants, sans autre modification du dispositif.

La présente invention a été décrite en référence à un mode de réalisation avantageux de celle-ci, mais il est bien entendu qu'elle n'est pas limitée par les exemples représentés sur les dessins. Des modifications sont envisageables pour l'homme du métier sans sortir du cadre de la présente invention tel que défini par les revendications annexées.

## Revendications

1. Dispositif de distribution de produit fluide, comprenant :
- un organe de distribution de produit fluide (10), tel qu'une pompe ou une valve actionnée manuellement, ledit organe de distribution de produit fluide étant fixé par une bague de fixation (30) sur un réservoir (1),
- une tête d'actionnement (20), montée sur ledit organe de distribution et déplaçable pour actionner ledit organe de distribution,
- un élément de blocage amovible (40) qui, en position de blocage empêche l'actionnement de l'organe de distribution,
- au moins un élément sécable (50) solidaire de l'élément de blocage pour témoigner d'une première utilisation,
ledit élément de blocage étant assemblé autour de la bague de fixation (30), entre la tête (20) et le réservoir (1), et coopèrant, en position de blocage, avec ladite tête d'actionnement et ledit réservoir pour empêcher une déplacement axial de ladite tête d'actionnement par rapport audit réservoir, **caractérisé en ce que** ledit au moins un élément sécable (50), avant la première utilisation, entoure au moins partiellement ledit élément de blocage (40).

2. Dispositif selon la revendication 1, dans lequel ledit élément sécable (50) est clipsé sur ledit élément de blocage (40) lors de son assemblage.

3. Dispositif selon la revendication 2, dans lequel ledit élément de blocage (40) comporte au moins une ouverture (46), adaptée à recevoir au moins un élément d'enclipsage (51) dudit élément sécable (50).

4. Dispositif selon l'une quelconque des revendications précédentes, dans lequel l'élément de blocage comporte au moins une projection (41) s'étendant radialement vers l'intérieur dudit élément de blocage.

5. Dispositif selon l'une quelconque des revendications précédentes, dans lequel l'élément de blocage (40) comporte une bague ouverte (45) élastiquement déformable.

6. Dispositif selon la revendication 5, dans lequel l'élément de blocage (40) et ledit au moins un élément sécable (50) forment une bague fermée, avant la première utilisation.

7. Dispositif selon l'une quelconque des revendications précédentes, dans lequel ledit au moins un élément sécable est adapté à se casser lorsqu'il est séparé de l'élément de blocage.

8. Dispositif selon les revendications 5 à 7, dans lequel ledit au moins un élément sécable est formé par une barrette (50) reliant les deux côtés de ladite bague ouverte (45), avant la première utilisation.

9. Dispositif selon la revendication 8, dans lequel ladite barrette, avant la première utilisation :
- est, au niveau d'une première extrémité (57), solidaire d'un premier desdits deux côtés de ladite bague ouverte,
- s'étend autour de la partie de la paroi latérale de ladite bague de fixation non encerclée par ladite bague ouverte,
- et coopère, au niveau d'une deuxième extrémité (55), avec le deuxième desdits deux côtés de ladite bague ouverte.

10. Dispositif selon les revendications 7 à 9, dans lequel ladite barrette se casse au niveau de la liaison solidaire (57) entre ladite barrette et ladite bague ouverte (45).

11. Dispositif selon l'une quelconque des revendications précédentes, dans lequel l'élément de blocage est maintenu en position de blocage par des moyens de retenue, lesdits moyens de retenue étant déformables élastiquement.

12. Dispositif selon la revendication 11, dans lequel lesdits moyens de retenue sont formés par ladite bague ouverte (45) et/ou au moins une projection (41).

13. Dispositif selon l'une quelconque des revendications précédentes, dans lequel ledit élément de blocage comporte des moyens de préhension (43) pour l'encliquetage et le désencliquetage de ladite bague ouverte (45).

## Patentansprüche

1. Ausgabevorrichtung für ein fluides Produkt, umfassend:
- eine Ausgabeeinrichtung für ein fluides Produkt (10), wie eine Pumpe oder ein manuell betätigtes Ventil, wobei die Ausgabeeinrichtung für ein fluides Produkt durch einen Befestigungsring (30) auf einem Behälter (1) befestigt ist,
- einen Betätigungskopf (20), der auf der Ausgabeeinrichtung montiert und durch Betätigung der Ausgabeeinrichtung verschiebbar ist,
- ein abnehmbares Sperrelement (40), welches in der Sperrposition die Betätigung der Ausgabeeinrichtung verhindert,
- mindestens ein teilbares Element (50) in einem Stück mit dem Sperrelement, um einen ersten Gebrauch zu zeigen,
wobei das Sperrelement um den Befestigungsring (30) zwischen dem Kopf (20) und dem Behälter (1) montiert ist und in der Sperrposition mit dem Betätigungskopf und dem Behälter zusammenwirkt, um eine axiale Verschiebung des Betätigungskopfes in Bezug auf den Behälter zu verhindern, **dadurch gekennzeichnet, dass** das mindestens eine teilbare Element (50) das Sperrelement (40) vor dem ersten Gebrauch zumindest teilweise umgibt.

2. Vorrichtung nach Anspruch 1, wobei das teilbare Element (50) bei seiner Montage auf das Sperrelement (40) aufgeklammert wird.

3. Vorrichtung nach Anspruch 2, wobei das Sperrelement (40) mindestens eine Öffnung (46) umfasst, die dazu geeignet ist, mindestens ein Klammerelement (51) des teilbaren Elements (50) aufzunehmen.

4. Vorrichtung nach einem der vorhergehenden Ansprüche, wobei das Sperrelement mindestens einen Vorsprung (41) umfasst, der sich radial ins Innere des Sperrelements erstreckt.

5. Vorrichtung nach einem der vorhergehenden Ansprüche, wobei das Sperrelement (40) einen elastisch verformbaren offenen Ring (45) umfasst.

6. Vorrichtung nach Anspruch 5, wobei das Sperrelement (40) und das mindestens eine teilbare Element (50) vor dem ersten Gebrauch einen geschlossenen Ring bilden.

7. Vorrichtung nach einem der vorhergehenden Ansprüche, wobei das mindestens eine teilbare Element dazu geeignet ist, zu brechen, wenn es von dem Sperrelement getrennt wird.

8. Vorrichtung nach den Ansprüchen 5 bis 7, wobei das mindestens eine teilbare Element durch einen Steg (50) gebildet ist, der die beiden Seiten des offenen Rings (45) vor dem ersten Gebrauch verbindet.

9. Vorrichtung nach Anspruch 8, wobei der Steg vor dem ersten Gebrauch:
- an einem ersten Ende (57) mit einer ersten der beiden Seiten des offenen Rings einstückig ist,
- sich um den Teil der Seitenwand des Befestigungsrings erstreckt, der nicht von dem offenen Ring umgeben ist,
- und an einem zweiten Ende (55) mit der zweiten der beiden Seiten des offenen Rings zusammenwirkt.

10. Vorrichtung nach den Ansprüchen 7 bis 9, wobei der Steg an der einstückigen Verbindung (57) zwischen dem Steg und dem offenen Ring (45) bricht.

11. Vorrichtung nach einem der vorhergehenden Ansprüche, wobei das Sperrelement durch Haltemittel in der Sperrposition gehalten wird, wobei die Haltemittel elastisch verformbar sind.

12. Vorrichtung nach Anspruch 11, wobei die Haltemittel durch den offenen Ring (45) und/oder mindestens einen Vorsprung (41) gebildet sind.

13. Vorrichtung nach einem der vorhergehenden Ansprüche, wobei das Sperrelement Greifmittel (43) zum Einrasten und Ausrasten des offenen Rings (45) umfasst.

## Claims

1. A fluid dispenser device comprising:
a fluid dispenser member (10) such as a manually-actuated pump or valve, said fluid dispenser member being fastened by a fastener ring (30) on a reservoir (1);
an actuator head (20) that is mounted on said dispenser member, and that is movable so as to actuate said dispenser member;
• a removable blocking element (40) that, in its blocking position, prevents the dispenser member from being actuated; and
• at least one breakable element (50) that is secured to the blocking element so as to indicate first use;
said blocking element being assembled around the fastener ring (30), between the head (20) and the reservoir (1), and, in its blocking position, cooperating with said actuator head and said reservoir so as to prevent said actuator head from moving axially relative to said reservoir, the device being **characterized in that** said at least one breakable element (50) surrounds said blocking element (40), at least in part, before first use.

2. A device according to claim 1, wherein said breakable element (50) is clipped on said blocking element (40) while it is being assembled.

3. A device according to claim 2, wherein said blocking element (40) includes at least one opening (46) that is adapted to receive at least one clip element (51) for clipping said breakable element (50).

4. A device according to any preceding claim, wherein the blocking element includes at least one projection (41) that extends radially towards the inside of said blocking element.

5. A device according to any preceding claim, wherein the blocking element (40) comprises an open ring (45) that is elastically deformable.

6. A device according to claim 5, wherein the blocking element (40) and said at least one breakable element (50) form a closed ring before first use.

7. A device according to any preceding claim, wherein said at least one breakable element is adapted to break when it is separated from the blocking element.

8. A device according to claims 5 to 7, wherein said at least one breakable element is formed by a strip (50) that interconnects the two sides of said open ring (45) before first use.

9. A device according to claim 8, wherein, before first use, said strip:
• at a first end (57), has a solid connection to a first of said two sides of said open ring;
• extends around the portion of the side wall of said fastener ring that is not encircled by said open ring; and
• at a second end (55), co-operates with the second of said two sides of said open ring.

10. A device according to claims 7 to 9, wherein said strip breaks at the solid connection (57) between said strip and said open ring (45).

11. A device according to any preceding claim, wherein the blocking element is held in its blocking position by retaining means, said retaining means being elastically deformable.

12. A device according to claim 11, wherein said retaining means are formed by said open ring (45) and/or by at least one projection (41).

13. A device according to any preceding claim, wherein said blocking element includes grip means (43) for snap-fastening and unfastening said open ring (45).
